# DEMANDE DE BREVET EUROPEEN

(11) **EP 0 611 559 A1**
(43) Date de publication de la demande: **24.08.1994**
(21) Numéro de dépôt: 93440021.9
(22) Date de dépôt: 18.02.1993
(51) Int. Cl.: A61F 2/38

(54) **Prothèse de genou unicondylienne**

(71) Demandeur: SOCIETE CIVILE ESSOR, F-74540 Perrignier (FR)
(72) Inventeur: Perringerard, Yvan, F-51100 Reims (FR)
(74) Mandataire: Polus, Camille

(57) **Abrégé**

Prothèse de genou unicondylienne composée d'un implant fémoral et d'un implant tibial.

L'implant fémoral (1) comporte une berge (12) sur le périmètre de sa face (11) en contact avec l'os ainsi que des plots (13) inclinés à 30° dans sa partie distale (10), son sommet (14) étant affiné et arrondi et ne dépassant pas la limite trochléo-distale, et sa partie postérieure (15) présentant une surface plane (16) correspondant à la coupe osseuse ; tandis que l'implant tibial est constitué d'une embase tibiale (2) sur laquelle est fixé un patin (3) en matière plastique, l'embase tibiale (2) étant constituée d'une plaque (20) en forme de demi-cercle prolongée, perpendiculairement à son plan, sur son côté rectiligne, par un aileron d'ancrage (21), la périphérie de la face de la plaque (20) en contact avec l'os comportant en outre une berge susceptible, comme la berge (12) de l'implant fémoral (1), de recevoir un revêtement améliorant sa fixation.

## Description

La présente invention a pour objet une prothèse de genou unicondylienne destinée à remplacer les surfaces articulaires fémorale et tibiale du genou, et composée d'un implant fémoral et d'un implant tibial.

Parmi les implants fémoraux connus à ce jour, certains, dits de "resurfaçage", sont appliqués sur la surface articulaire préalablement préparée. Toutefois ce type d'implant présente fréquemment l'inconvénient d'une mauvaise tenue dans la partie osseuse, qu'il risque en outre d'endommager.

D'autre part, les implants tibiaux existants ne permettent généralement pas de réaliser une fixation primaire rapide de l'embase tibiale qui en fait partie, non plus que d'assurer la résistance de cette dernière aux forces de cisaillement en rotation.

La présente invention a pour but de remédier à ces divers inconvénients des prothèses de genou connues à ce jour en proposant une prothèse unicondylienne composée d'un implant fémoral de resurfacage et d'un implant tibial formé d'une embase tibiale sur laquelle est fixé un patin constituant la surface de frottement.

Afin de présenter une bonne tenue dans la partie osseuse sans risque d'endommager outre mesure cette dernière lors de sa mise en place, et afin de ne pas entrer en conflit avec la rotule lors des mouvements de flexion, l'implant fémoral de la prothèse selon l'invention présente les caractéristiques suivantes :
- d'une part, sa partie interne comprend une face plane postérieure et une face courbe antérieure munie de deux plots inclinés à environ 30°, et comportant sur son pourtour une berge d'une hauteur de l'ordre de 3/10 mm susceptible de recevoir un revêtement améliorant sa fixation ;
- d'autre part, le sommet de la partie antérieure dudit implant est affiné et arrondi et ne dépasse pas la limite trochléo-distale, tandis que sa partie postérieure possède une épaisseur lui permettant de limiter au maximum la résection de l'os et présente une surface plane correspondant à la coupe osseuse.

Le revêtement améliorant la fixation dudit implant fémoral peut consister soit en un revêtement favorisant la repousse osseuse, comme par exemple une couche de microbilles ou une couche d'hydroxyapatite, soit en un revêtement favorisant la fixation par un ciment acrylique.

L'implant tibial de la prothèse selon l'invention comporte une embase tibiale sur laquelle est fixé par tout moyen approprié un patin en matière plastique, et de préférence en polyéthylène, constituant la surface de frottement.

Conformément à l'invention, en vue de pouvoir assurer une fixation primaire rapide de l'embase tibiale, et afin qu'elle résiste aux forces de cisaillement en rotation, ladite embase consiste en une plaque approximativement en forme de demi-cercle prolongée, perpendiculairement à son plan, sur son côté rectiligne, par un aileron d'ancrage antirotatoire, et comportant en outre, sur sa face en contact avec l'os, une berge périphérique susceptible de recevoir un revêtement destiné à améliorer sa fixation.

Toujours conformément à l'invention, le patin en polyéthylène présente une courbure antéro-postérieure, mais pas de courbure dans le plan frontal, afin d'éviter l'autocentrage du condyle dans le plateau tibial, ce qui pourrait entraîner des contraintes sur l'implant et sur le condyle sain.

Encore conformément à l'invention,la face interne du patin est dépouillée à 10° afin d'eviter les conflits avec la coupe osseuse médiane antéro-postérieure, lors de la mise en place de l'implant tibial.

L'embase tibiale de la prothèse selon l'invention peut être fixée à l'os soit à l'aide d'un ciment approprié, soit à l'aide de vis, auquel cas elle comporte au moins deux orifices pour le passage de vis.

Les caractéristiques et les avantages de la présente invention ressortiront plus clairement de la description qui suit et qui se rapporte au dessin annexé, lequel en représente un mode de réalisation non limitatif.

Dans le dessin annexé :
- la figure 1a représente une vue de l'implant fémoral d'une prothèse selon l'invention, vu en coupe partielle selon la ligne X X' de la figure 1b.
- la figure 1b représente une vue du même implant, vu selon la flèche F₁ de la figure 1a.
- la figure 1c représente une vue du même implant, vu selon la flèche F₂ de la figure 1b.
- la figure 2a représente une vue de dessus de l'embase tibiale d'une prothèse selon l'invention.
- la figure 2b représente une vue en coupe de la même embase tibiale, selon la ligne Y Y' de la figure 2a.
- la figure 3a représente une vue de dessus du patin tibial d'une prothèse selon l'invention.
- la figure 3b représente une vue du même patin tibial, vu selon la flèche F₃ de la figure 3a.
- la figure 3c représente une vue en coupe, selon la ligne Z Z' de la figure 3a.

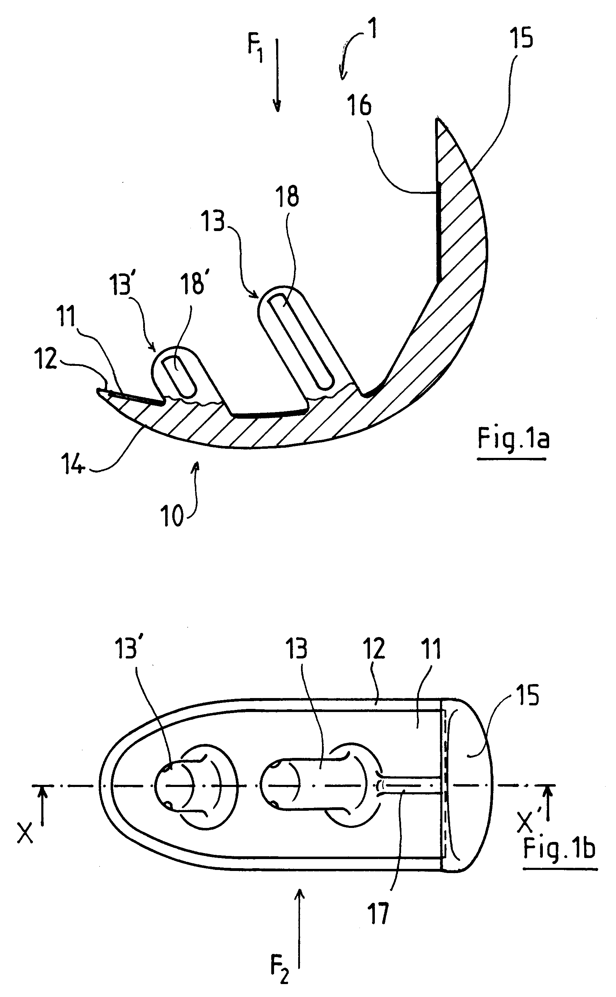

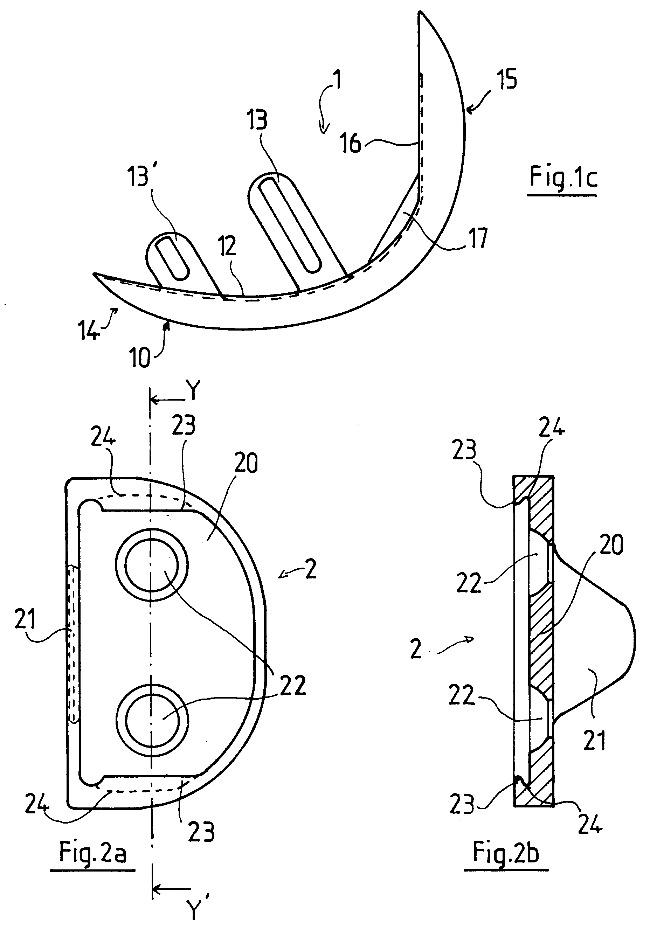

Si l'on se réfère aux figures 1a, 1b et 1c on peut voir qu'un implant fémoral 1 selon l'invention comprend une partie distale 10 présentant la courbure anatomique du condyle distal fémoral, dont la face 11 en contact avec l'os comporte une berge 12 de 3/10 mm délimitant une surface susceptible de recevoir un revêtement favorisant la repousse osseuse, ou un revêtement favorisant la fixation par un ciment acrylique. La face 11 comporte en outre deux plots 13, 13' inclinés à 30° pour assurer la fixation et l'orientation de l'implant fémoral 1, l'inclinaison à 30° permettant de transmettre à la pièce fémorale des forces de compression lors de la flexion du genou.

Le sommet 14 de la partie distale 10 affiné et il est arrondi afin d'éviter d'éventuels conflits avec la rotule lors des mouvements de flexion. Une fois posé, il ne doit dépasser en aucun cas la limite trochléo-distale du genou.

La partie postérieure 15 de l'implant fémoral 1 a une épaisseur de l'ordre de 6 millimètres afin de limiter au maximum la résection de l'os. Elle présente une surface plane interne 16 correspondant à la coupe osseuse et participant à la fixation mécanique.

Les deux parties, distale 10 et postérieure 15 de l'implant fémoral 1 sont reliées entre elles par une arête arrondie 17 renforçant la tenue mécanique de la pièce.

Si l'on se réfère aux figures 2a et 2b on peut voir qu'une embase tibiale 2 comprend une plaque 20 en forme de demi-cercle munie de deux orifices 22 pour le passage de vis et un aileron d'ancrage antirotatoire 21 perpendiculaire au plan de la plaque 20, biseauté afin de pouvoir être impacté dans la partie osseuse, assurant ainsi une fixation primaire immédiate de ladite embase.

Les orifices 22 permettent une pose réversible de l'implant tibial, qui s'adapte indifféremment sur le côté nterne ou externe tant du genou droit que du genou gauche.

Les orifices 22 présentent un profil en cuvette où se loge la tête d'une vis et autorisent un débattement de 15° de ladite vis permettant de l'insérer dans la partie corticale de l'os, partie la plus dure et la plus résistante. La vis favorise un effet "press-fit" appuyant l'embase 2 contre la coupe osseuse.

La face en contact avec l'os comporte à sa périphérie une berge (non représentée sur les figures) de 3/10 mm qui délimite un emplacement pour un revêtement accélérant la repousse osseuse.

La face recevant le patin en polyéthylène comporte un rebord périphérique 23 et deux gorges 24 permettant la fixation dudit patin par clipsage.

Dans un autre mode de réalisation de l'implant tibial, l'embase peut être fixée à l'os par un ciment acrylique couvrant la surface de contact avec l'os. Dans ce cas l'embase ne comporte pas d'orifices pour le passage des vis.

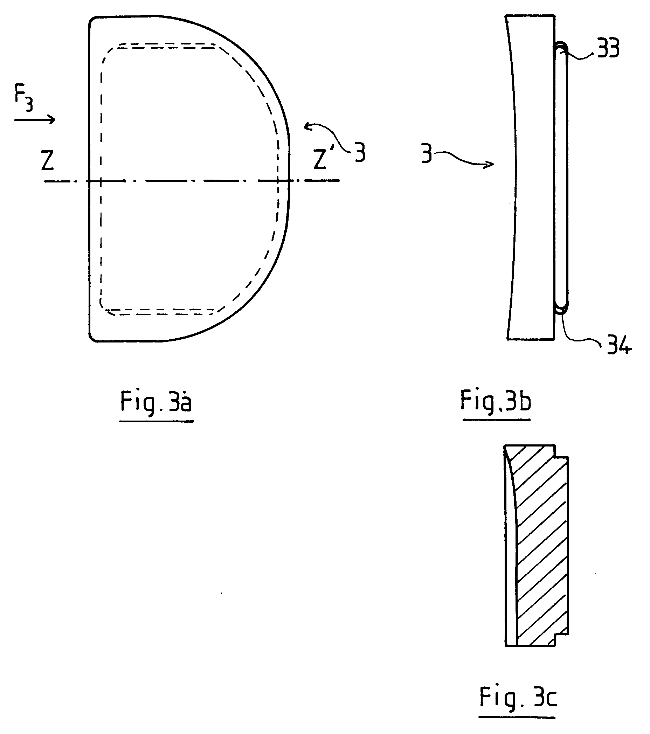

Si l'on se réfère aux figures 3a, 3b, 3c on peut voir un patin 3 en polyéthylène, présentant une courbure antéro-postérieure mais ne présentant pas de courbure dans le plan frontal. Du côté destiné à venir au contact de l'embase 2 le patin 3 comporte une base 33 de diamètre inférieur, munie de deux bourrelets 34 destinés à être insérés dans les gorges 24 de l'embase 2 en vue de permettre la fixation du patin 3 sur l'embase 2 par simple clipsage.

Les pièces fémorale et tibiale de la prothèse selon l'invention peuvent bien entendu être réalisées en plusieurs tailles adaptables aux morphologies des genoux à appareiller.

D'autre part, il va de soi que la présente invention ne saurait être limitée à la description qui précède d'un de ses modes de réalisation, susceptible de subir un certain nombre de modifications sans pour autant sortir du cadre de l'invention.

## Revendications

1. Prothèse de genou unicondylienne composée d'un implant fémoral et d'un implant tibial, caractérisée en ce que l'implant fémoral (1) comporte une berge (12) sur le périmètre de sa face (11) en contact avec l'os ainsi que des plots (13) inclinés à 30° dans sa partie distale (10), son sommet (14) étant affiné et arrondi et ne dépassant pas la limite trochléo-distale, et sa partie postérieure (15) présentant une surface plane (16) correspondant à la coupe osseuse ; tandis que l'implant tibial est constitué d'une embase tibiale (2) sur laquelle est fixé un patin (3) en matière plastique, l'embase tibiale (2) étant constituée d'une plaque (20) en forme de demi-cercle prolongée, perpendiculairement à son plan, sur son côté rectiligne, par un aileron d'ancrage (21), la périphérie de la face de la plaque (20) en contact avec l'os comportant en outre une berge susceptible, comme la berge (12) de l'implant fémoral (1), de recevoir un revêtement améliorant sa fixation.

2. Prothèse de genou selon la revendication 1, caractérisée en ce que le patin (3) présente une courbure antéro-postérieure, mais pas de courbure dans le plan frontal.

3. Prothèse de genou selon les revendications 1 et 2, caractérisée en ce que la face interne du patin (3) est dépouillée à 10°.

4. Prothèse de genou selon l'une quelconque des revendications précédentes, caractérisée en ce que le patin (3) est en polyéthylène.

5. Prothèse de genou selon l'une quelconque des revendications 1 à 4, caractérisée en ce que l'embase tibiale (2) est munie de deux orifices (22) pour le passage de vis.

6. Prothèse de genou selon l'une quelconque des revendications 1 à 4, caractérisée en ce que l'embase tibiale (2) est fixée à l'os par un ciment acrylique.

7. Prothèse selon l'une quelconque des revendications précédentes, caractérisée en ce que la face interne de l'embase tibiale (2) comporte un rebord périphérique (23) et deux gorges (24) permettant la fixation du patin (3) par clipsage.

8. Prothèse selon la revendication 1, caractérisée en ce que le revêtement améliorant sa fixation est un revêtement favorisant la repousse osseuse.

9. Prothèse selon la revendication 1, caractérisée en ce que le revêtement améliorant sa fixation est un revêtement favorisant la fixation par un ciment acrylique.
